(19) Europäisches Patentamt / European Patent Office / Office européen des brevets

(11) EP 1 792 982 B1

(12) **EUROPÄISCHE PATENTSCHRIFT**

(45) Veröffentlichungstag und Bekanntmachung des Hinweises auf die Patenterteilung:
**11.04.2012 Patentblatt 2012/15**

(51) Int Cl.:
*C12N 9/16* (2006.01) *C12N 15/81* (2006.01) *C12N 15/69* (2006.01) *C12N 15/55* (2006.01) *C12N 1/19* (2006.01)

(21) Anmeldenummer: **06021699.1**

(22) Anmeldetag: **21.07.2001**

(54) **Codon-optimierte alkalisch Phosphatase und ihre Expression in Hefe**

Codon-optimised alkaline phosphatase and its expression in yeast

Phosphatase alkaline à codons optimisés et son expression dans la levure

(84) Benannte Vertragsstaaten:
**AT BE CH CY DE DK ES FI FR GB GR IE IT LI LU MC NL PT SE TR**

(30) Priorität: **25.07.2000 DE 10036491**

(43) Veröffentlichungstag der Anmeldung:
**06.06.2007 Patentblatt 2007/23**

(62) Dokumentnummer(n) der früheren Anmeldung(en) nach Art. 76 EPÜ:
**01117822.5 / 1 176 205**

(73) Patentinhaber:
- **Roche Diagnostics GmbH 68305 Mannheim (DE)**
  Benannte Vertragsstaaten:
  **DE**
- **F. Hoffmann-La Roche AG 4070 Basel (CH)**
  Benannte Vertragsstaaten:
  **AT BE CH CY DK ES FI FR GB GR IE IT LI LU MC NL PT SE TR**

(72) Erfinder:
- **Mueller, Rainer 82377 Penzberg (DE)**
- **Thalhofer, Johann-Peter 82362 weilheim (DE)**
- **Geipel, Frank 82377 Penzberg (DE)**
- **Hoelke, Werner 82377 Penzberg (DE)**
- **Glaser, Stephan 82402 Seeshaupt (DE)**
- **Eckstein, Hellmut 82362 Weilheim (DE)**
- **Kirschbaum, Thomas 82393 Iffeldorf (DE)**
- **Bommarius, Bettina Atlanta, GA 30327 (US)**

(56) Entgegenhaltungen:
**EP-A- 0 700 997 EP-A- 0 955 369**
**WO-A-01/66693 GB-A- 2 200 118**

- **BATARD YANNICK ET AL: "Increasing expression of P450 and P450-reductase proteins from monocots in heterologous systems." ARCHIVES OF BIOCHEMISTRY AND BIOPHYSICS, Bd. 379, Nr. 1, 1. Juli 2000 (2000-07-01), Seiten 161-169, XP001027387 ISSN: 0003-9861**
- **LI HONG ET AL: "The relation between codon usage, base correlation and gene expression level in Escherichia coli and yeast." JOURNAL OF THEORETICAL BIOLOGY, Bd. 181, Nr. 2, 1996, Seiten 111-124, XP001055854 ISSN: 0022-5193**
- **DATABASE PUBLIC MEDLINE NCBI; PMID: 11059269 Mai 2000 (2000-05), ZHAO X, ET AL.: "Synonymous codon usage in Pichia Pastoris" XP002191950 & SHENG WU KUNG CH ENG HSUEH PAO, Bd. 16, Nr. 3, Mai 2000 (2000-05), Seiten 308-311,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

SEQUENCE LISTING

<110> Roche Diagnostics GmbH
<120> Expression of alkaline phosphatase in yeast
<130> 5387/00/
<140>
<141>
<160> 38
<170> Patent In Ver. 2.1
<210> 1
<211> 1476
<212> DNA
<213> Bovine
<400> 1

```
gaattcctca tcccagctga ggaggaaaac cccgccttct ggaaccgcca ggcagcccag 60
gcccttgatg tagccaagaa gttgcagccg atccagacag ctgccaagaa tgtcatcctc 120
ttcttggggg atgggatggg ggtgcctacg gtgacagcca ctcggatcct aaaggggcag 180
atgaatggca aactgggacc tgagacaccc ctggccatgg accagttccc atacgtggct 240
ctgtccaaga catacaacgt ggacagacag gtgccagaca gcgcaggcac tgccactgcc 300
tacctgtgtg ggtcaaggg caactacaga accatcggtg taagtgcagc cgcccgctac 360
aatcagtgca acacgacacg tgggaatgag gtcacgtctg tgatcaaccg ggccaagaaa 420
gcagggaagg ccgtgggagt ggtgaccacc accagggtgc agcatgcctc cccagccggg 480
gcctacgcgc acacggtgaa ccgaaactgg tactcagacg ccgacctgcc tgctgatgca 540
cagaagaatg gctgccagga catcgccgca cagctggtct acaacatgga tattgacgtg 600
atcctgggtg gaggccgaat gtacatgttt cctgaggggga ccccagaccc tgaataccca 660
gatgatgcca gtgtgaatgg agtccggaag gacaagcaga acctggtgca ggaatggcag 720
gccaagcacc agggagccca gtatgtgtgg aaccgcactg cgctccttca ggcggccgat 780
gactccagtg taacacacct catgggcctc tttgagccgg cagacatgaa gtataatgtt 840
cagcaagacc acaccaagga cccgaccctg gcggagatga cggaggcggc cctgcaagtg 900
ctgagcagga acccccgggg cttctacctc ttcgtggagg gaggccgcat tgaccacggt 960
caccatgacg gcaaagctta tatggcactg actgaggcga tcatgtttga caatgccatc 1020
gccaaggcta acgagctcac tagcgaactg gacacgctga tccttgtcac tgcagaccac 1080
tcccatgtct tctcttttgg tggctacaca ctgcgtggga cctccatttt cggtctggcc 1140
cccggcaagg ccttagacag caagtcctac acctccatcc tctatggcaa tggcccaggc 1200
tatgcgcttg gcgggggctc gaggcccgat gttaatggca gcacaagcga ggaaccctca 1260
taccggcagc aggcggccgt gcccctggct agcgagaccc acgggggcga agacgtggcg 1320
gtgttcgcgc gaggcccgca ggcgcacctg gtgcacggcg tgcaggagga gaccttcgtg 1380
gcgcacatca tggcctttgc gggctgcgtg gagccctaca ccgactgcaa tctgccagcc 1440
cccgccaccg ccaccagcat ccccgactag ggtacc 1476
```

<210> 2
<211> 40
<212> DNA
<213> Artificial Sequence
<220>
<223> Description of Artificial Sequence: Artificial
<400> 2
gcgcgaattc ctcatcccag ctgaggagga aaaccccgcc 40
<210> 3
<211> 36

<212> DNA
<213> Artificial Sequence
<220>
<223> Description of Artificial Sequence: Artificial
<400> 3
cgcgggtacc ctagtcgggg atgctggtgg cggtgg 36
<210> 4
<211> 487
<212> PRT
<213> Bovine
<400> 4

```
Leu Ile Pro Ala Glu Glu Glu Asn Pro Ala Phe Trp Asn Arg Gln Ala
1               5                   10                  15

Ala Gln Ala Leu Asp Val Ala Lys Lys Leu Gln Pro Ile Gln Thr Ala
            20                  25                  30

Ala Lys Asn Val Ile Leu Phe Leu Gly Asp Gly Met Gly Val Pro Thr
        35                  40                  45

Val Thr Ala Thr Arg Ile Leu Lys Gly Gln Met Asn Gly Lys Leu Gly
    50                  55                  60

Pro Glu Thr Pro Leu Ala Met Asp Gln Phe Pro Tyr Val Ala Leu Ser
65                  70                  75                  80

Lys Thr Tyr Asn Val Asp Arg Gln Val Pro Asp Ser Ala Gly Thr Ala
                85                  90                  95

Thr Ala Tyr Leu Cys Gly Val Lys Gly Asn Tyr Arg Thr Ile Gly Val
            100                 105                 110

Ser Ala Ala Ala Arg Tyr Asn Gln Cys Asn Thr Thr Arg Gly Asn Glu
        115                 120                 125

Val Thr Ser Val Ile Asn Arg Ala Lys Lys Ala Gly Lys Ala Val Gly
    130                 135                 140

Val Val Thr Thr Thr Arg Val Gln His Ala Ser Pro Ala Gly Ala Tyr
145                 150                 155                 160

Ala His Thr Val Asn Arg Asn Trp Tyr Ser Asp Ala Asp Leu Pro Ala
                165                 170                 175

Asp Ala Gln Lys Asn Gly Cys Gln Asp Ile Ala Ala Gln Leu Val Tyr
            180                 185                 190

Asn Met Asp Ile Asp Val Ile Leu Gly Gly Gly Arg Met Tyr Met Phe
        195                 200                 205

Pro Glu Gly Thr Pro Asp Pro Glu Tyr Pro Asp Asp Ala Ser Val Asn
    210                 215                 220

Gly Val Arg Lys Asp Lys Gln Asn Leu Val Gln Glu Trp Gln Ala Lys
225                 230                 235                 240
```

```
His Gln Gly Ala Gln Tyr Val Trp Asn Arg Thr Ala Leu Leu Gln Ala
                245                 250                 255

Ala Asp Asp Ser Ser Val Thr His Leu Met Gly Leu Phe Glu Pro Ala
            260                 265                 270

Asp Met Lys Tyr Asn Val Gln Gln Asp His Thr Lys Asp Pro Thr Leu
        275                 280                 285

Ala Glu Met Thr Glu Ala Ala Leu Gln Val Leu Ser Arg Asn Pro Arg
    290                 295                 300

Gly Phe Tyr Leu Phe Val Glu Gly Gly Arg Ile Asp His Gly His His
305                 310                 315                 320

Asp Gly Lys Ala Tyr Met Ala Leu Thr Glu Ala Ile Met Phe Asp Asn
                325                 330                 335

Ala Ile Ala Lys Ala Asn Glu Leu Thr Ser Glu Leu Asp Thr Leu Ile
            340                 345                 350

Leu Val Thr Ala Asp His Ser His Val Phe Ser Phe Gly Gly Tyr Thr
        355                 360                 365

Leu Arg Gly Thr Ser Ile Phe Gly Leu Ala Pro Gly Lys Ala Leu Asp
    370                 375                 380

Ser Lys Ser Tyr Thr Ser Ile Leu Tyr Gly Asn Gly Pro Gly Tyr Ala
385                 390                 395                 400

Leu Gly Gly Gly Ser Arg Pro Asp Val Asn Gly Ser Thr Ser Glu Glu
                405                 410                 415

Pro Ser Tyr Arg Gln Gln Ala Ala Val Pro Leu Ala Ser Glu Thr His
            420                 425                 430

Gly Gly Glu Asp Val Ala Val Phe Ala Arg Gly Pro Gln Ala His Leu
        435                 440                 445

Val His Gly Val Gln Glu Glu Thr Phe Val Ala His Ile Met Ala Phe
    450                 455                 460

Ala Gly Cys Val Glu Pro Tyr Thr Asp Cys Asn Leu Pro Ala Pro Ala
465                 470                 475                 480

Thr Ala Thr Ser Ile Pro Asp
                485
```

<210> 5
<211> 1476
<212> DNA
<213> Artificial Sequence
<220>
<223> Description of Artificial Sequence: Artificial
<400> 5

```
gaattcttga ttccagctga agaagaaaat ccagcttttt ggaatagaca agctgctcaa 60
gctttggatg ttgctaagaa gttgcaacca attcaaactg ctgctaagaa tgttattttg 120
tttttgggtg atggtatggg tgttccaact gttactgcta ctagaatttt gaagggtcaa 180
atgaatggta agttgggtcc agaaactcca ttggctatgg atcaatttcc atacgttgct 240
ttgtctaaga cttacaatgt tgatagacaa gttccagatt ctgctggtac tgctactgct 300
tacttgtgtg gtgttaaggg taattacaga actattggtg tttctgctgc tgctagatac 360
aatcaatgta atactactag aggtaatgaa gttacttctg ttattaatag agctaagaag 420
gctggtaagg ctgttggtgt tgttactact actagagttc aacatgcttc tccagctggt 480
gcttacgctc atactgttaa tagaaattgg tactctgatg ctgatttgcc agctgatgct 540
caaaagaatg gttgtcaaga tattgctgct caattggttt acaatatgga tattgatgtt 600
attttgggtg gtggtagaat gtacatgttt ccagaaggta ctccagatcc agaataccca 660
gatgatgctt ctgttaatgg tgttagaaag gataagcaaa atttggttca agaatggcaa 720
gctaagcatc aaggtgctca atatgtttgg aatagaactg ctttgttgca agctgctgat 780
gattctagtg ttactcattt gatgggtttg tttgaaccag ctgatatgaa gtataatgtt 840
caacaagatc atactaagga tccaactttg gctgaaatga ctgaagctgc tttgcaagtt 900
ttgtctagaa atccaagagg tttttacttg tttgttgaag gtggtagaat tgatcatggt 960
catcatgatg gtaaggctta tatggctttg actgaagcta ttatgtttga taatgctatt 1020
gctaaggcta atgaattgac ttctgaattg gatactttga ttttggttac tgctgatcat 1080
agtcatgttt tttcttttgg tggttacact ttgagaggta cttctatttt tggtttggct 1140
ccaggtaagg ctttggatag taagtcttac acttctattt tgtatggtaa tggtccaggt 1200
tatgctttgg gtggtggttc tagaccagat gttaatggta gtactagtga agaaccatct 1260
tacagacaac aagctgctgt tccattggct agtgaaactc atggtggtga agatgttgct 1320
gtttttgcta gaggtccaca agctcatttg gttcatggtg ttcaagaaga aacttttgtt 1380
gctcatatta tggcttttgc tggttgtgtt gaaccataca ctgattgtaa tttgccagct 1440
ccagctactg ctactagtat tccagattaa ggtacc                           1476
```

<210> 6
<211> 78
<212> DNA
<213> Artificial Sequence
<220>
<223> Description of Artificial Sequence: Artificial
<400> 6

```
gcgcgaattc ttgattccag ctgaagaaga aaatccagct tttggaata gacaagctgc 60
tcaagctttg gatgttgc                                             78
```

<210> 7
<211> 70
<212> DNA
<213> Artificial Sequence
<220>
<223> Description of Artificial Sequence: Artificial
<400> 7

```
ccaaaaacaa aataacattc ttagcagcag tttgaattgg ttgcaacttc ttagcaacat 60
ccaaagcttg                                                       70
```

<210> 8
<211> 69
<212> DNA
<213> Artificial Sequence
<220>
<223> Description of Artificial Sequence: Artificial
<400> 8

gaatgttatt ttgtttttgg gtgatggtat gggtgttcca actgttactg ctactagaat 60

tttgaaggg 69

<210> 9
<211> 70
<212> DNA
<213> Artificial Sequence
<220>
<223> Description of Artificial Sequence: Artificial
<400> 9

ggaaattgat ccatagccaa tggagtttct ggacccaact taccattcat ttgacccttc 60
aaaattctag 70

<210> 10
<211> 71
<212> DNA
<213> Artificial Sequence
<220>
<223> Description of Artificial Sequence: Artificial
<400> 10

gctatggatc aatttccata cgttgctttg tctaagactt acaatgttga tagacaagtt 60
ccagattctg c 71

<210> 11
<211> 71
<212> DNA
<213> Artificial Sequence
<220>
<223> Description of Artificial Sequence: Artificial
<400> 11

ccaatagttc tgtaattacc cttaacacca cacaagtaag cagtagcagt accagcagaa 60
tctggaactt g 71

<210> 12
<211> 72
<212> DNA
<213> Artificial Sequence
<220>
<223> Description of Artificial Sequence: Artificial
<400> 12

gtaattacag aactattggt gtttctgctg ctgctagata caatcaatgt aatactacta 60
gaggtaatga ag 72

<210> 13

<211> 74
<212> DNA
<213> Artificial Sequence
<220>
<223> Description of Artificial Sequence: Artificial
<400> 13

agtaacaaca ccaacagcct taccagcctt cttagctcta ttaataacag aagtaacttc 60
attacctcta gtag 74

<210> 14
<211> 74
<212> DNA
<213> Artificial Sequence
<220>
<223> Description of Artificial Sequence: Artificial
<400> 14

gctgttggtg ttgttactac tactagagtt caacatgctt ctccagctgg tgcttacgct 60
catactgtta atag 74

<210> 15
<211> 68
<212> DNA
<213> Artificial Sequence
<220>
<223> Description of Artificial Sequence: Artificial
<400> 15

caaccattct tttgagcatc agctggcaaa tcagcatcag agtaccaatt tctattaaca 60
gtatgagc 68

<210> 16
<211> 55
<212> DNA
<213> Artificial Sequence
<220>
<223> Description of Artificial Sequence: Artificial
<400> 16 gatgctcaaa agaatggttg tcaagatatt gctgctcaat tggtttacaa tatgg 55
<210> 17
<211> 72
<212> DNA
<213> Artificial Sequence
<220>
<223> Description of Artificial Sequence: Artificial
<400> 17

ccttctggaa acatgtacat tctaccacca cccaaaataa catcaatatc catattgtaa 60
accaattgag ca 72

<210> 18
<211> 71
<212> DNA
<213> Artificial Sequence

<220>
<223> Description of Artificial Sequence: Artificial
<400> 18

gtacatgttt ccagaaggta ctccagatcc agaataccca gatgatgctt ctgttaatgg 60

tgttagaaag g 71

<210> 19
<211> 73
<212> DNA
<213> Artificial Sequence
<220>
<223> Description of Artificial Sequence: Artificial
<400> 19

catattgagc accttgatgc ttagcttgcc attcttgaac caaattttgc ttatcctttc 60
taacaccatt aac 73

<210> 20
<211> 71
<212> DNA
<213> Artificial Sequence
<220>
<223> Description of Artificial Sequence: Artificial
<400> 20

gcatcaaggt gctcaatatg tttggaatag aactgctttg ttgcaagctg ctgatgattc 60
tagtgttact c 71

<210> 21
<211> 54
<212> DNA
<213> Artificial Sequence
<220>
<223> Description of Artificial Sequence: Artificial
<400> 21
cttcatatca gctggttcaa acaaacccat caaatgagta acactagaat catc 54
<210> 22
<211> 59
<212> DNA
<213> Artificial Sequence
<220>
<223> Description of Artificial Sequence: Artificial
<400> 22 gaaccagctg atatgaagta taatgttcaa caagatcata ctaaggatcc aactttggc 59
<210> 23
<211> 67
<212> DNA
<213> Artificial Sequence
<220>
<223> Description of Artificial Sequence: Artificial
<400> 23

cctcttggat ttctagacaa aacttgcaaa gcagcttcag tcatttcagc caaagttgga 60
tccttag 67

<210> 24
<211> 69
<212> DNA
<213> Artificial Sequence
<220>
<223> Description of Artificial Sequence: Artificial
<400> 24

gtctagaaat ccaagaggtt tttacttgtt tgttgaaggt ggtagaattg atcatggtca 60
tcatgatgg 69

<210> 25
<211> 73
<212> DNA
<213> Artificial Sequence
<220>
<223> Description of Artificial Sequence: Artificial
<400> 25

ccttagcaat agcattatca aacataatag cttcagtcaa agccatataa gccttaccat 60
catgatgacc atg 73

<210> 26
<211> 74
<212> DNA
<213> Artificial Sequence
<220>
<223> Description of Artificial Sequence: Artificial
<400> 26

gataatgcta ttgctaaggc taatgaattg acttctgaat tggatacttt gattttggtt 60
actgctgatc atag 74

<210> 27
<211> 73
<212> DNA
<213> Artificial Sequence
<220>
<223> Description of Artificial Sequence: Artificial
<400> 27

ccaaaccaaa aatagaagta cctctcaaag tgtaaccacc aaaagaaaaa acatgactat 60
gatcagcagt aac 73

<210> 28
<211> 73
<212> DNA
<213> Artificial Sequence
<220>

<223> Description of Artificial Sequence: Artificial
<400> 28

cttctatttt tggtttggct ccaggtaagg ctttggatag taagtcttac acttctattt 60
tgtatggtaa tgg 73

<210> 29
<211> 76
<212> DNA
<213> Artificial Sequence
<220>
<223> Description of Artificial Sequence: Artificial
<400> 29

ctagtactac cattaacatc tggtctagaa ccaccaccca aagcataacc tggaccatta 60
ccatacaaaa tagaag 76

<210> 30
<211> 77
<212> DNA
<213> Artificial Sequence
<220>
<223> Description of Artificial Sequence: Artificial
<400> 30

gatgttaatg gtagtactag tgaagaacca tcttacagac aacaagctgc tgttccattg 60
gctagtgaaa ctcatgg 77

<210> 31
<211> 73
<212> DNA
<213> Artificial Sequence
<220>
<223> Description of Artificial Sequence: Artificial
<400> 31

caccatgaac caaatgagct tgtggacctc tagcaaaaac agcaacatct tcaccaccat 60
gagtttcact agc 73

<210> 32
<211> 74
<212> DNA
<213> Artificial Sequence
<220>
<223> Description of Artificial Sequence: Artificial
<400> 32

gctcatttgg ttcatggtgt tcaagaagaa acttttgttg ctcatattat ggcttttgct 60
ggttgtgttg aacc 74

<210> 33
<211> 82

<212> DNA
<213> Artificial Sequence
<220>
<223> Description of Artificial Sequence: Artificial
<400> 33

gcgcggtacc ttaatctgga atactagtag cagtagctgg agctggcaaa ttacaatcag 60
tgtatggttc aacacaacca gc 82

<210> 34
<211> 31
<212> DNA
<213> Artificial Sequence
<220>
<223> Description of Artificial Sequence: Artificial
<400> 34
gcgcgcctag gagatctaac atccaaagac g 31
<210> 35
<211> 29
<212> DNA
<213> Artificial Sequence
<220>
<223> Description of Artificial Sequence: Artificial
<400> 35
cgcgcgctag cggatccgca caaacgaag 29
<210> 36
<211> 10
<212> PRT
<213> Saccharomyces cerevisiae
<400> 36

Glu Ala Glu Ala Glu Phe Leu Ile Pro Ala
1 5 10

<210> 37
<211> 4
<212> PRT
<213> Saccharomyces cerevisiae
<400> 37

Leu Ile Pro Ala
1

<210> 38
<211> 6
<212> PRT
<213> Saccharomyces cerevisiae
<400> 38

Glu Ala Glu Ala Glu Phe
1 5

## Beschreibung

Die Erfindung betrifft ein Verfahren zu rekombinanten Herstellung bzw. Expression von eukaryontischer Alkalischer Phosphatase. Die Erfindung betrifft darüber hinaus eine codon-optimierte DNA, kodierend für eine eukaryontische hochaktive Alkalische Phosphatase mit einer spezifischen Aktivität über 3000 U/mg. Die Erfindung betrifft ferner ein Verfahren zur Insertion der DNA in einen Vektor zur Expression in Hefe-Zellen.

Alkalische Phosphatasen (AP) sind dimere, zinkhaltige, nichtspezifische Phosphomonoesterasen, die sowohl in prokaryontischen wie auch eukaryontischen Organismen, z.B. in E. coli und Säugern vorkommen (McComb et al., 1979 Alkaline Phosphatases Plenum Press, New York). Der Vergleich der Primärstruktur verschiedener Alkalischer Phosphatasen ergab einen hohen Homologiegrad (25-30% Homologie zwischen E. coli- und Säuger-AP; Millàn, 1988 Anticancer Res. 8, 995-1004; Harris, 1989 Clin. Chim. Acta 186, 133-150).

Im Menschen und höheren Tieren besteht die AP-Familie aus vier Mitgliedern, die in verschiedenen Genloci codiert sind (Millan, 1988 Anticancer Res. 8, 995-1004; Harris 1989 Clin. Chim. Acta 186,133-150). Zur Familie der Alkalischen Phosphatasen zählen die gewebespezifischen APs (Placenta-AP (PLAP), Germzellen-AP (GCAP) und Darm-AP (IAP)) und die nicht-gewebespezifischen APs (TnAP), die vorwiegend in Leber, Niere und Knochen lokalisiert sind.

Eine entscheidende Eigenschaft der bislang bekannten APs ist die große Variabilität in der katalytischen Aktivität der Säuger-APs, die einen 10-100fach höheren $k_{cat}$s-Wert besitzen als E. coli-AP. Unter den Säuger-APs zeigen die APs aus dem Rinderdarm (bIAP) die höchsten spezifischen Aktivitäten. Diese Eigenschaft machen die bIAPs attraktiv für biochemische Anwendungen wie z.B. der Einsatz entsprechender Enzymkonjugate als diagnostisches Reagenz oder zur Dephosphorylierung von DNA. Die Existenz verschiedener Alkalischer Phosphatasen aus dem Rinderdarm mit unterschiedlichen hohen spezifischen Aktivitäten ist in EP 0 955 369 bzw. Manes et al. (1998), J. Biol. Chem. 273 No. 36, 23353-23360, beschrieben. Bislang ist eine rekombinante Expression von eukaryontischen niederaktiven (bis 3000 U/mg) Alkalischen Phosphatasen in verschiedenen eukaryontischen Zelllinien wie z.B. CHO-Zellen (bIAP I/WO 93/18139; Weissig et al. 1993, Biochem J. 260, 503-508), COS-Zellen (humane Placenta-AP/Berger et al., 1987 Biochemistry 84, 4885-4889) oder Baculovirus-Expressionssystem (humane Placenta-AP/Davis et al. 1992, Biotechnology 10, 1148-1150) beschrieben, Auch ist die Expression von höher aktiven AP's (spez. Aktivität >3000 U/mg) aus dem Rinderdarm in CHO-Zellen beschrieben (bIAP II, III und IV/Manes et al. 1998, J. Biol. Chem. 273 No. 36, 23353-23360). Nachteil der Expression von Alkalischen Phosphatasen in diesen Expressionssystemen ist jedoch die geringe Expressionsleistung, die die rekombinante Herstellung, insbesondere einer hochaktiven AP nicht wirtschaftlich macht.

Eine Expression von eukaryontischen Alkalischen Phosphatasen in prokaryontischen Expressionswirten wie z.B. E. coli ist zwar prinzipiell möglich (humane Placenta-AP/Beck and Burtscher, 1994 Protein Expression and Purification 5, 192-197), jedoch weisen die in Prokaryonten exprimierten Alkalischen Phosphatasen keine Glykosylierung auf, die insbesondere bei der Herstellung von. Enzymkonjugaten essentiell ist.

Demzufolge liegt der vorliegenden Erfindung die Aufgabe zugrunde, ein widerstandsfähiges und stabiles Expressionsverfahren zur Herstellung glykosylierter eukaryontischer Alkalischer Phosphatase mit hoher spezifischer Aktivität zu entwickeln, das zudem auf Grund hoher Expressionsleistung eine wirtschaftliche Herstellung einer entsprechenden Alkalischen Phosphatase ermöglicht und darüber hinaus ein Enzym liefert, das bezüglich seiner Eigenschaften wie z.B. spezifischer Aktivität und Thermostabilität mit der nativen hochaktiven oder niederaktiven Alkalischen Phosphatase (kommerziell erhältlich z.B. bei Roche Diagnostics GmbH, Biozyme, Oriental Yeast) vergleichbar ist.

Ein erster Gegenstand der vorliegenden Erfindung ist ein Transformierter Wirtsstamm, erhältlich durch die Durchführung der Schritte (a) Klonieren einer codon-optimierten Gensequenz codierend für eine eukaryontische Alkalische Phosphatase in jeweils einem ersten Expressionsvektor mit einem Resistenzgen gegen ein erstes Antibiotikum und einem zweiten Expressionsvektor mit einem Resistenzgen gegen ein zweites Antibiotikum; gefolgt von (b) Transformieren eines Hefe-Wirts mit dem ersten Expressionsvektor und Selektion von Transformanten, die mindestens eine Kopie des ersten Expressionsvektors mit der Gensequenz und dem Resistenzgen gegen das erste. Antibiotikum in das Genom integriert haben, durch Wachstum auf Nährmedium mit einer geringen Konzentration des ersten Antibiotikums; gefolgt von (c) Erhöhen der Genkopienzahl des ersten Expressionsvektors durch Mehrfachtransformation mit dem ersten Expressionsvektor, wobei durch Wachstum auf Nährmedium mit einer im Vergleich zu Schritt (b) höheren Konzentration des ersten Antibiotikums selektioniert wird; gefolgt von (d) Transformieren von transformierten Klonen des Wirts mit der höchsten Expressionsleistung an Alkalischer Phosphatase mit dem zweiten Expressionsvektor und Selektion von Transformanten, die neben den Kopien des ersten Expressionsvektors Kopien des zweiten Expressionsvektors mit der Gensequenz und dem Resistenzgen gegen das zweite Antibiotikum aufgenommen haben, durch Wachstum auf Nährmedium mit dem zweitem Antibiotikum; gefolgt von; (e) Erhöhen der Genkopienzahl des zweiten Expressionsvektors durch Mehrfachtransformation mit dem zweiten Expressionsvektor, wobei durch Wachstum auf Nährmedium mit einer im Vergleich zu Schritt (d) höheren Konzentration des zweiten Antibiotikums selektioniert wird; gefolgt von (f) Selektionieren der Klone, die mehrere Kopien der Gensequenz und der Selektionsmarker-Resistenzgene stabil in das Genom integriert haben; und (g) Untersuchen der in Schritt (f) erhaltenen Klone auf erhöhte Expression durch Aktivitätstest der Alkalischen

Phosphatase; wobei die Expressionsleistung der in Schritt (t) erhaltenen Klone um den Faktor 4 gegenüber der Expression der in Schritt (b) erhaltenen Klone gesteigert ist.

Ein weiterer Gegenstand der Erfindung ist eine Flüssigkultur in einem Kulturbehälter enthaltend ein Nährmedium, Methanol und einen erfindungsgemäßen transformierten Wirtsstamm.

Ein weiterer Gegenstand der Erfindung ist eine Biomasse enthaltend einen erfindungsgemäßen transformierten Wirtsstamm.

Weiterhin offenbart dieses Dokument ein Verfahren zur Herstellung einer eukaryontischen Alkalischen Phosphatase mit hoher spezifischer Aktivität in Hefe, insbesondere in einer methylotrophen Hefe umfassend die Schritte:

a) Klonierung einer Gensequenz in unterschiedlichen Vektoren
b) Transformation der Hefe,
c) Expression und
d) Reinigung der Alkalischen Phosphatase,
dadurch gekennzeichnet, daß

(i) ein erster Vektor ein Resistenzgen gegen einen ersten Selektionsmarker aufweist,
(ii) Transformanten, die das Resistenzgen und die Gensequenz in das Genom integriert haben, durch Wachstum auf Nährmedium mit einer geringen Konzentration an erstem Selektionsmarker selektioniert werden,
(iii) die Genkopienzahl durch Mehrfachtransformation erhöht wird, wobei Mehrfachtransformanten durch Wachstum auf Nährmedium mit erhöhtem Selektionsdruck selektioniert werden,
(iv) ein zweiter Vektor, welcher neben der Gensequenz ein Resistenzgen gegen einen zweiten Selektionsmarker aufweist, zugegeben wird,
(v) die Genkopienzahl durch Mehrfachtransformation mit dem zweiten Vektor erhöht wird, wobei Mehrfachtransformanten durch Wachstum auf Nährmedium mit erhöhtem Selektionsdruck selektioniert werden, und
(vi) die Klone selektioniert werden, die mehrere Kopien der Gensequenz und der Selektionsmarker-Resistenzgene stabil in das Genom integriert haben.

Bevorzugt ist als Gensequenz eine DNA-Sequenz, die für eine eukaryontische alkalische Phosphatase codiert, welche eine spezifische Aktivität von mehr als 3000 U/mg, in besonderen Fällen von über 7000 U/mg bis etwa 10.000 U/mg aufweist. Beispielsweise hat sich erfindungsgemäß eine DNA-Sequenz gemäß SEQ ID NO: 1 als geeignet erwiesen. Besonders bevorzugt wird eine codon-optimierte DNA-Sequenz, die auf Aminosäure-Ebene der Gensequenz SEQ ID NO: 1 entspricht. Codon-Optimierung bedeutet, daß durch stumme Mutation, d.h. Änderungen auf DNA-Ebene, die sich jedoch nicht auf Aminosäure-Ebene auswirken, jedes Codon beispielsweise von SEQ ID NO: 1 zur Steigerung der Translation nach den Bedürfnissen des ausgewählten Expressionswirtes optimiert wird, wodurch beispielsweise die Gensequenz gemäß SEQ ID NO: 5 erhalten wird. Es können jedoch auch andere Gensequenzen als SEQ ID NO:1, die für Alkalische Phosphatasen codieren und gegebenenfalls codon-optimiert sind, in den Vektor eingebaut werden, wie z.B. bIAPI, III, IV (DE 198 19 962 bzw. EP 0 955 369). Besonders bevorzugt für das erfindungsgemäße Verfahren ist die Verwendung einer codon-optimierten Gensequenz gemäß SEQ ID NO: 5. Die entsprechende Gensequenz wird dann in einen oder mehrere Vektor(en) kloniert, welche(r) entsprechend dem zu transformierenden Wirt ausgewählt wird bzw. werden.

Als Hefe-Wirt sind besonders methylotrophe Hefen, z.B. Hefe Pichia pastoris, Hansenula polymorpha, aber auch andere Hefen, wie z.B. Saccharomyces cerevisiae, Yarrowia lipolytica oder Schizosaccharomyces pombe geeignet. Geeignete Vektoren sind dem Fachmann bekannt, wie beispielsweise pPICZαA, pPIC9K, Yes-Vektoren, pTEF1/Zeo, pYDI (z.B. Invitrogen). Der so entstandene Expressionsvektor wird bevorzugt in verschiedene Stämme von Pichia pastoris transformiert und stabil in das Genom integriert werden. Die stabile Integration in das Hefegenom hat den Vorteil, daß bei der späteren Produktion der beispielsweise eukaryontischen, hochaktiven Alkalischen Phosphatase in großvolumigen Fermenten kein Selektionsdruck benötigt wird. Stabile Integration in das Genom bedeutet, daß der Expressionsvektor über homologe Rekombination in das Genom von z.B. Pichia pastoris eingebaut wird und somit als fester Bestandteil des Hefegenoms von Generation zu Generation weitervererbt wird (Cregg, J.M. et al., Mol. Cell. Biol. 5 (1985), 3376-3385).

Die Erhöhung der Genkopienzahl in der methylotrophen Hefe wurde durch Mehrfachtransformation bei gleichzeitiger Erhöhung des Selektionsdruckes mit einem geeigneten Selektionsmarker, z.B. einem Antibiotikum wie beispielsweise Zeocin® bzw. Geneticin (G418) oder einem Auxotrophiemarker erreicht, wonach nur Klone lebensfähig sind, die mehrere Kopien des Expressionsvektors stabil in das Genom integriert haben. Um gegen höhere Konzentrationen des als Selektionsmarker verwendeten Antibiotikums resistent zu sein, ist es erforderlich, daß die Klone vermehrt Resistenzprotein produzieren. Dies kann beispielsweise durch eine multiple Integration des Expressionsvektors erfolgen, der neben der Expressionskassette für die beispielsweise hochaktive Alkalische Phosphatase auch das Resistenzgen für das als Selektionsmärker verwendete Antibiotikum enthält.

Die Aufgabe eukaryontische Alkalische Phosphatase in einem widerstandsfähigen und stabilen Expressionsverfahren mit hoher Expressionsleistung wirtschaftlich herzustellen, konnte erst durch die Kombination der Maßnahmen (i) bis (vi) des hier offenbarten Verfahrens erreicht werden. So führte beispielsweise die Transformation eines Pichia pastoris Stammes X-33 mit einen Expressionsvektor, der das bIAPII-Gen gemäß SEQ ID NO: 1 enthält, ohne diese Maßnahmen nicht zum gewünschten Erfolg (siehe Beispiel 1 und 2). Mit dem Verfahren konnte zwar die Expressionsleistung im Vergleich zu der Expression von bIAPII in CHO-Zellen (Manes et al., 1998 J. Biol. Chem, 273 No. 36, 23353-23360) erheblich gesteigert werden, jedoch erlaubt das Verfahren nicht die Herstellung einer rekombinanten Alkalischen Phosphatase unter wirtschaftlichen Gesichtspunkten.

Eine der erforderlichen Maßnahmen zur Bereitstellung des erfindungsgemäßen Verfahrens ist die Synthese einer codon-optimierten Gensequenz.Um jedes Codon zur Expression in Hefe zu optimieren war eine komplette de novo Synthese des ca. 1,5 kBp langen Gens, das für die eukaryontische hochaktive Alkalische Phosphatase kodiert, erforderlich. Durch Rückübersetzung der Aminosäuresequenz der eukaryontischen hochaktiven Alkalischen Phosphatase gemäß SEQ ID No. 4 (bIAP-II) konnte durch Ausnutzung des regenerierten Codes jedes Codon, wenn erforderlich, optimiert werden. Dazu wurde das Gen in 28 Oligonukleotide mit einer Länge von 54 bis 82 Nukleotide unterteilt. Die Oligonukleotide wurden als alternierende Folge von Sinnstrang- und Gegenstrangfragment entworfen, die jeweils mit ihren 5'- bzw. 3'-Enden mit den benachbarten Oligonukleotiden komplementär überlappten. Der Überlappungsbereich wurde jeweils so gewählt, daß bei der Annealingreaktion in der späteren PCR-Reaktion eine unspezifische Bindung weitgehend verhindert wird. Die Oligonukleotide am 5'- bzw. 3'-Ende des Gens wurden stromaufwärts bzw. stromabwärts des kodierenden Bereichs mit Erkennungsstellen für Restriktionsendonukleasen versehen, die für eine spätere Insertion des synthetischen Gens gemäß SEQ ID No. 5 in Expressionsvektoren genutzt werden konnten. So wurde stromaufwärts eine Erkennungsstelle für die Restriktionsendonuklease EcoRI und stromabwärts eine Erkennungsstelle für die Restriktionsendonuklease Asp718 eingebaut. Die Sequenzen der Oligonukleotide sind in SEQ ID No. 6 bis 33 dargestellt.

Die Gensynthese wurde mittels PCR-Reaktion durchgeführt. Dazu wurde der kodierende Bereich zunächst in drei Teilstücke unterteilt (Oligonukleotide 6 bis 15, 16 bis 23, 24 bis 33) und diese Teilstücke in getrennten PCR-Reaktionen erzeugt. Bei der Gensynthese mit überlappenden komplementären Oligonukleotiden mittels PCR-Reaktion erfolgt dabei ein schrittweises Verlängern des Genfragmentes bis zum volle Länge-Produkt, das dann in den weiteren Zyklen amplifiziert wird. Die Annealingtemperatur richtet sich dabei nach dem Überlappungsbereich mit der geringsten Schmelztemperatur.

Die drei Teilstücke wurden anschließend mittels Agarosegelelektrophorese analysiert, die Produkte mit der erwarteten Länge aus dem Gel mittels QIAquick Gel Extraction Kit (Qiagen) isoliert und in einer weiteren PCR-Reaktion zu dem kompletten Genprodukt synthetisiert. Die PCR-Reaktion wurde dabei in den ersten 5 Zyklen ohne Zugabe der Primern am 5'-Ende und am 3'-Ende des gesamten Gens durchgeführt, so daß zunächst aus den drei Teilstücken wenige Fragmente des Genproduktes der erwarteten Länge entstehen. Die Annealingtemperatur richtet sich dabei nach dem Überlappungsbereich mit der geringsten Schmelztemperatur. Danach wurden die endständigen Primer zugegeben und die Annealingtemperatur entsprechend der Annealingtemperatur des Primers mit der geringsten Schmelztemperatur erhöht. In weiteren 25 Zyklen wurde danach das Genfragment der erwarteten Länge hochamplifiziert.

Der PCR-Ansatz wurde mittels Agarosegelelektrophorese analysiert, das Genfragment mit der erwarteten Größe isoliert (QIAquick Gel Extraction Kit/Qiagen).

Die Klonierung eines entsprechenden PCR Fragmentes, die Transformation in Pichia pastoris sowie die Expression ist in Beispiel 3 beschrieben.

Mit dem Codon-optimierten Gen für die hochaktive Alkalische Phosphatase konnte die Expressionsleistung um den Faktor 3 gegenüber den ersten Versuchen mit dem Wildtypgen gesteigert werden.

Jedoch war mit diesen Klonen noch kein wirtschaftliches Verfahren zur Herstellung der hochaktiven Alkalischen Phosphatase erreicht.

Eine Maßnahme zur Steigerung der Expressionsleistung heterologer und homologer Proteine in Pichia pastoris ist die Erhöhung der Genkopienzahl in der Zelle durch Mehrfachtransformation. Diese Maßnahme kann die Erhöhung des Transkriptionsproduktes, der mRNA des Zielgens, dienen. Man erreicht die Erhöhung der Genkopienzahl durch Mehrfachtransformation eines Klones mit dem Expressionsvektor bei gleichzeitiger Erhöhung des Selektionsdruckes beim anschließenden Wachstum der Transformanten auf Nährplatten mit erhöhter Konzentration des als Selektionsmarker verwendeten Antibiotikums. Dabei wird ein Expressionsklon, der aus dem ersten Transformationsdurchgang bereits mindestens eine Kopie des Expressionsvektors aufgenommen haben, wieder kompetent gemacht (s. Beispiel 1) und wieder mit Expressionsvektor transformiert. Selektioniert werden Transformanten, die mehrere Kopien des Expressionsvektors in das Genom integriert haben, durch Ausplattieren auf Nährplatten mit höherem Selektionsdruck, d.h. Platten mit einer höheren Konzentration des als Selektionsmarker verwendeten Antibiotikums (z.B. Zeocin®), als beim ersten Transformationsdurchgang. Dabei wird zunächst die höchste Konzentration des als Selektionsmarker verwendeten Antibiotikums, bei der die Klone aus dem ersten Transformationsdurchgang noch wachsen können, ermittelt und demzufolge die Konzentration des als Selektionsmarker verwendeten Antibiotikums in den YPDS-Agarplatten nach der zusätzlichen Transformation über den ermittelten Schwellenwert erhöht. Durch die Erhöhung der Kopienzahl des Expressionsvektors wird auch die Kopienzahl des Resistenzgens, das Bestandteil des Expressionsvektors ist, erhöht und damit auch die Resistenz gegen höhere Konzentrationen des als Selektionsmarker verwendeten Antibiotikums erreicht. Durch Variation der Konzentration des als Selektionsmarker verwendeten Antibiotikums in den Nährplatten (ca. 100 bis 2000 μg/ml) können auch Klone mit unterschiedlich hohen Kopienzahlen des Expressionsvektors im Genom selektioniert werden (s. Beispiel 4).

Eine weitere Maßnahme zur Steigerung der Expressionsleistung heterologer und homologer Proteine in Hefe, wie beispielsweise Pichia pastoris ist die Erhöhung der Genkopienzahl durch Mehrfachselektion. Man erreicht dies durch Transformation eines Expressionsklons, der bereits durch Mehrfachtransformation mit einem Expressionsvektor, der die Expressionskassette mit dem Zielgen (z.B. das Gen, das für die hochaktive Alkalische Phosphatase kodiert gemäß SEQ ID NO: 5) und ein Resistenzgen für das als Selektionsmarker verwendete erste Antibiotikum (z.B. Zeocin®) enthält, optimiert wurde, mit einem zweiten Expressionsvektor, der das Zielgen (z.B. das Gen, das für die hochaktive Alkalische Phosphatase kodiert gemäß SEQ ID NO: 5) und ein Resistenzgen für das als Selektionsmarker verwendete zweite Antibiotikum (z.B. Geneticin (G418)) enthält. Beim anschließenden Ausplattieren der Transformanten auf Nährplatten, die das zweite Antibiotikum als Selektionsmarker enthalten, werden nur Klone selektioniert, die neben den Kopien des Expressionsvektors mit Resistenzgen für das als Selektionsmarker verwendete erste Antibiotikum auch mindestens eine Kopie des Expressionsvektors mit Resistenzgen für das als Selektionsmarker verwendete zweite Antibiotikum aufgenommen haben. Diese Expressionsklone können nun wiederum einer weiteren Mehrfachtransformation mit dem Expressionsvektor mit Resistenzgen für das als Selektionsmarker verwendete zweite Antibiotikum unterzogen werden (s. Beispiel 5).

Durch die Kombination der Maßnahmen der Mehrfachtransformation und der Doppelselektion konnte die Expressionsleistung um den Faktor 4 gegenüber der Expressionsleistung der Klonen aus dem ersten Transformantendurchgang mit den codon-optimierten Gen gesteigert werden.

Die rekombinante Alkalische Phosphatase kann durch Extraktionsmethoden, die dem Fachmann prinzipiell bekannt sind, aus der Biomasse extrahiert werden z.B. "Protein Purification", Springer-Verlag, Herausg. Robert Scopes (1982). Durch chromatographische Trennungsmethoden, wie insbesondere unter Verwendung von hydrophoben Säulenmaterialien und eines Kationenaustauschers, wird ein bandenreines Produkt mit einer spezifischen Aktivität von mehr als 7000 U/mg erzielt.

Um die rekombinante hochaktive Alkalische Phosphatase zu charakterisieren wurde das gereinigte Produkt einer N-terminalen Sequenzierung unterworfen.

Es wurde dominant die Sequenz EAEAEFLIPA (SEQ ID NO: 36) bestimmt. Die Sequenz korreliert eindeutig mit der N-terminalen Sequenz der AP "LIPA" (SEQ ID NO: 37) und dem Linkerpeptid des Konstruktes EAEAEF (SEQ ID NO: 38), das durch die Klonierungsstrategie der Gensequenz in den Vektor und durch die Abspaltung des α-Faktor-Signalpeptides durch eine Kex2-Signalpeptidase (z.B. Invitrogen) ensteht.

Die Stabilität der rekombinanten Alkalischen Phosphatase Produktes wurde im Vergleich zu der natürlich vorkommenden Alkalischen Phosphatase untersucht. Die Proben ergeben, bei einer thermischen Belastung (55°C) der Lösung vergleichbare Ergebnisse.

Mit der vorliegenden Erfindung wird somit erstmals ein Verfahren beschrieben, daß eine wirtschaftliche Herstellung einer rekombinanten Alkalischen Phosphatase aus Säugerzellen, wie z.B. Rinderdarm ermöglicht, die vergleichbare Eigenschaften zur nativen hochaktiven Alkalischen Phosphatase aus dem Rinderdarm besitzt und glykosyliert ist.

Des weiteren wird mit der vorliegenden Erfindung eine DNA Sequenz beschrieben gemäß SEQ ID NO: 5 als codon-optimierte Gensequenz für die Expression des Gens der hochaktiven Alkalischen Phosphatase Pichia pastoris.

Mit der Erfindung wird des weiteren ein Vektor enthaltend SEQ ID NO: 5 offenbart, besonders bevorzugt ist ein Vektor pHAP10-3 gemäß Fig. 2. pHAP10-3 ist der Vektor pPICZαA, der kommerziell erhältlich ist (Invitrogen), und der das erfindungsgemäße Gen gemäß SEQ ID NO: 5 enthält, das unter Kontrolle des AOX 1-Promotors steht.

Des weiteren ist Gegenstand ein erfindungsgemäßer Wirtsstamm, der mit den erfindungsgemäßen Vektoren transformiert wurde. Besonders bevorzugt ist der Pichia pastoris X-33 Stamm transformiert mit Vektor pHAP10-3.

Des weiteren bevorzugt ist ein Vektor, der die gesamte Expressionskassette aus pHAP 10-3 enthält, die im wesentlichen aus dem AOX 1-Promotor, dem Signalpeptid des α-Paktors aus Saccharomyces cerevisiae, das im korrekten Leserahmen hinter das Signalpeptid kloniert codon-optimierte Zielgen gemäß SEQ ID NO: 5, das für die hochaktive Alkalische Phosphatase kodiert und der AOX 1-Transkriptionsterminationsregion besteht (siehe Fig. 3). Besonders bevorzugt ist der Vektor pHAP 10-3/9K, der aus dem kommerziell erhältlichen Vektor pPIC9K (Invitrogen) und der Expressionskassette aus pHAP 10-3, einschließlich des synthetischen Gens gemäß SEQ ID NO: 5 besteht.

Die Vektoren pHAP 10-3 und pHAP 10-3/K sind gleichermaßen relevant, da der letztendliche Produktionsklon Kopien von beiden Vektoren enthält.

Gegenstand der Erfindung ist des weiteren ein erfindungsgemäßen Wirtsstamm, der pHAP10-3/9K-Vektor transformiert wurde. Geeignet im Sinne der vorliegenden Erfindung sind jedoch auch andere dem Fachmann bekannte Vektoren und Stämme, wie beispielsweise YES-Vektoren, pYD1, pTEF 1/ZEO (Invitrogen) und Saccharomyces cerevisiae, Schizosaccharmyces pombe, Hansenula polymorpha, Yarrowia lipolytica und insbesondere Pichia pastoris X-33. Insbesondere ist erfindungsgemäß der Pichia pastoris X-33 Stamm transformiert mit dem Vektor pHAP10-3/9K bevorzugt.

Weiterhin wird mit der Erfindung ein Verfahren offenbart, zur Herstellung einer eurkaryontischen hochaktiven Alkalischen Phosphatase durch Expression des Proteins in einem erfindungsgemäßen Wirtsstam, der mit einem oder mehrerem erfindungsgemäßen Vektor(en), insbesondere mit dem pHAP10-3 Vektor bzw. pHAP10-3/9K Vektor transformiert wurde. Pichia pastoris-Stämme, die mit erfindungsgemäßen Vektoren transformiert wurden, sind für das erfindungsgemäße Verfahren besonders bevorzugt. Insbesondere bevorzugt ist dabei der Stamm Pichia pastoris X-33, der mit einem pHAP 10-3- und einem pHAP 10-3/9K-Vektor transformiert ist.

<u>Abbildungen</u>

Abbildung 1

Plasmidkarte von Expressionsvektor pHAP-1 mit dem bIAPII-Gen in pICZαA (Invitrogen).

Abbildung 2

Plasmidkarte von Expressionsvektor pHAP10-3 mit dem synthetischen Gen in pPIC9K (Invitrogen).

Abbildung 3

Plasmidkarte von Expressionsvektor pHAP10-3/9K mit dem synthetischen Gen in pPIC9K (Invitrogen).

<u>Abkürzungen</u>

| | |
|---|---|
| YPD: | Yeast Peptone Dextrose |
| YPDS: | Yeast Peptone Dextrose Sorbitol |
| BMGY: | Buffered Glyerol-complex medium |
| BMMY: | Buffered Methanol-complex medium |

<u>Beispiel 1:</u>

<u>Klonierung des bIAPII-Gens</u>

Das bIAPII-Gen gemäß SEQ ID No. 1 (EP 0 955 369;Manes et al., 1998, J. Biol. Chem. 273 No. 36, 23353-23360) wurde zunächst mittels PCR und Wahl geeigneter Primer gemäß SEQ ID NO: 2 und 3 stromaufwärts und stromabwärts mit für die Klonierung in Expressionsvektoren für Pichia pastoris geeigneten Restriktionsendonukleaseschnittstellen versehen. So wurde stromaufwärts die Restriktionsendonukleaseschnittstelle für EcoRI und stromabwärts die Restriktionsendonukleaseschnittstelle Asp718 I angehängt.

Das PCR-Fragment wurde mit EcoRI und Asp718 I (Roche Diagnostics GmbH) nachgeschnitten, nochmals isoliert (QIAquick Gel Extraction Kit/Qiagen) und anschließend in ein mit EcoRI und Asp718 I (Roche Diagnostics GmbH) linearisiertes und isoliertes (QIAquick Gel Extraction Kit/Qiagen) Vektorfragment des Expressionsvektors pPICZαA (Invitrogen) ligiert. In diesem Vektor steht das bIAPII-Gen unter Kontrolle des AOX 1-Promotors (Promotor für die Alkoholoxidase 1 aus Pichia pastoris, mit Methanol induzierbar und wird im korrekten Leserahmen hinter das Signalpeptid des α-Faktors aus Saccharomyces cerevisiae kloniert. Das so insertierte Genfragment wurde dann mittels Restriktionsanalyse und Sequenzierung auf fehlerfreie Basensequenz untersucht. Der so entstandene Expressionsvektor, der das bIAPII-Gen enthält, das für die eukaryontische hochaktive Alkalische Phosphatase kodiert, wurde pHAP-1 (s. Fig. 1) genannt.

<u>Transformation von pHAP-1 in Pichia pastoris</u>

Zur Transformation von pHAP-1 in Pichia pastoris X-33 mit anschließender Integration in das Genom wurde der Vektor zunächst mit SacI (Roche Diagnostics GmbH) linearisiert. Die Transformation wurde mittels Elektroporation mit dem Gene Pulser II (Biorad) durchgeführt.

Dazu wurde eine Kolonie von Pichia pastoris Wildtypstamm in 5 ml YPD-Medium (Invitrogen) angeimpft und bei 30°C über Nacht unter Schütteln inkubiert. Die Übernachtkultur wurde anschließend 1:2000 in 200 ml frisches YPD-Medium (Invitrogen) überimpft und über Nacht bei 30°C unter Schütteln inkubiert, bis eine $OD_{600}$ von 1,3-1,5 erreicht war. Die Zellen wurden abzentrifugiert (1500 x g / 5 Minuten) und das Pellet in 200 ml eiskaltem, sterilen Wasser (0°C) resuspendiert. Die Zellen wurden wiederum abzentrifugiert (1500 x g/5 Minuten) und in 100 ml eiskaltem, sterilen Wasser (0°C) resuspendiert. Die Zellen wurden wiederum abzentrifugiert, in 10 ml eiskaltem (0°C) 1 M Sorbitol (ICN) resuspendiert. Die Zellen wurden wiederum abzentrifugiert, in 0,5 ml eiskaltem (0°C) 1 M Sorbitol (ICN) resuspendiert. Die so gewonnenen Zellen wurden auf Eis gehalten und sofort zur Transformation eingesetzt.

80 μl der Zellen wurden mit ca. 1 μg linearisierter pHAP-1-Vektor-DNA versetzt und der gesamte Ansatz in eine eiskalte (0°C) Elektroporationsküvette transferiert und weitere 5 Minuten auf Eis inkubiert. Anschließend wurde die Küvette in den Gene Pulser II (Biorad) überführt und die Transformation bei 1 kV, 1 kΩ und 25μF durchgeführt. Nach der Elektroporation wurde der Ansatz mit 1 ml 1M Sorbitol (ICN) versetzt und anschließend 100 bis 150 μl auf eine YPDS-Agarplatte (Invitrogen) mit 100μg/ml Zeocin® (Invitrogen) ausplattiert. Die Platten wurden anschließend 2-4 Tage bei 30°C inkubiert.

Die Klone wurden auf Raster-MD (= minimal Dextrose)-Platten überimpft und weiter analysiert. Gewachsene Klone wurden gepickt, in 20 μl steriles Wasser resuspendiert, mit 17,5 U Lyticase (Roche Diagnostics GmbH) aufgeschlossen (1 Stunde, 37°C) und direkt mittels PCR auf die korrekte Integration der bIAPII-Expressionskassette untersucht.

Klone, die die komplette Expressionskassette bei der Transformation in das Genom integriert haben, wurden dann in Expressionsversuchen eingesetzt.

<u>Expression der hochaktiven Alkalischen Phosphatase</u>

Positive Klone wurden in 3 ml BMGY-Medium (Invitrogen) angeimpft und über Nacht bei 30°C unter Schütteln inkubiert. Anschließend wurden die OD bei 600 nm bestimmt und so in 10ml BMMY-Medium (Invitrogen) überimpft, daß eine $OD_{600}$ von 1 resultierte. Das BMMY-Medium (Invitrogen) enthält Methanol (Mallinckrodt Baker B.V.), das die Expression der hochaktiven Alkalischen Phosphatase über den AOX 1-Promotor induziert.

Die Schüttelkolben wurden bei 30°C unter Schütteln inkubiert, alle 24 Stunden Proben gezogen, die $OD_{600}$ bestimmt, ein Aktivitätstest auf Expression der hochaktiven Alkalischen Phosphatase durchgeführt und jeweils mit 0,5% Methanol (Mallinckrodt Baker B.V.) zur weiteren Induktion nachgefüttert. Die Expressionsversuche liefen über 96 Stunden.

<u>Beispiel 2:</u>

<u>Test auf Aktivität der hochaktiven Alkalischen Phosphatase</u>

Je 500 μl wurden der Expressionskultur gemäß Beispiel 1 entnommen, die $OD_{600}$ bestimmt und die Zellen abzentrifugiert. Der Überstand wurde aufbewahrt und das Zellpellet wurde in einer der $OD_{600}$ entsprechenden Menge Y-PER™ (50 bis 300 μl/Pierce) zur Lyse resuspendiert und 1 Stunde bei Raumtemperatur geschüttelt. Anschließend wurde das Lysat zur Abtrennung von Zelltrümmern abzentrifugiert (15000 x g/5 Minuten) und der Überstand in frische Reaktionsgefäße überführt. 5 μl des Lysates wurden dann im Aktivitätstest eingesetzt.

Der Aktivitätstest funktioniert nach folgendem Prinzip:

$$\text{4-Nitrophenylphosphat} + H_2O \xrightarrow{\text{AP}} \text{4-Nitrophenol} + P_i$$

Gemessen wird die Absorptionszunahme bei 405 nm.

3 ml Diethanolamin-Puffer (1 mol/L Diethanolamin (Merck) pH 9,8, 0,5 mmol/L $MgCl_2$ (Riedel de Haen)) wurden mit 50 μl 4- Nitrophenylphosphatlösung (0,67 Mol/L 4- Nitrophenylphosphat, Na-Salz (Roche Diagnostics GmbH)) versetzt und der Ansatz auf 37°C temperiert. Anschließend wurde die Reaktion durch Zugabe von 5 μl Lysat gestartet und die Absorptionsänderung bei 37°C über 3 Minuten bestimmt und daraus das ΔE/min berechnet.

Die Aktivität wurde dann nach folgender Formel berechnet:

$$\text{Aktivität} = \frac{3{,}10}{\varepsilon \times 0{,}005 \times 1} \times \Delta E/\text{min} \times \frac{1}{\text{Faktor x}} \quad [\text{U/ml Probelösung}]$$

$\varepsilon$ = 18,2 [1 x $mmol^{-1}$ x $cm^{-1}$]
Faktor x = Konzentrierungsfaktor nach Zellaufschluss

Analog wurde die Aktivität aus dem Mediumüberstand der Expressionskulturen bestimmt. Hier wurden ebenfalls die Reaktion mit 5 µl des Überstandes gestartet, jedoch wurden zusätzlich noch jeweils 0,5 mM $ZnCl_2$ zugegeben. Die Berechnung erfolgte dabei ohne Faktor x.

Beispiel 3:

Klonierung des PCR-Fragmentes aus der Gensynthese

Das PCR-Fragment wurde mit EcoRI und Asp718 (Roche Diagnostics GmbH) nachgeschnitten, nochmals isoliert (QIAquick Gel Extraction Kit/Qiagen) und anschließend in ein mit EcoRI und Asp718 (Roche Diagnostics GmbH) linearisiertes und isoliertes (QIAquick Gel Extraction Kit/Qiagen) Vektorfragment des Expressionsvektors pPICZαA (Invitrogen) ligiert. In diesem Vektor steht das synthetische Gen unter Kontrolle des AOX 1-Promotors (Promotor für die Alkoholoxidase 1 aus Pichia pastoris, mit Methanol (Mallinckrodt Baker B.V.) induzierbar) und wird im korrekten Leserahmen hinter das Signalpeptid des α-Faktors aus Saccharomyces cerevisiae kloniert. Das so insertierte Genfragment wurde dann mittels Restriktionsanalyse und Sequenzierung auf fehlerfreie Basensequenz untersucht. Der so entstandene Expressionsvektor, der ein synthetisches Gen, das für die eukaryontische hochaktive Alkalische Phosphatase kodiert, enthält wurde pHAP10-3 (s. Fig. 2) genannt.

Transformation von pHAP10-3 in Pichia pastoris

Zur Transformation von pHAP10-3 in Pichia pastoris X-33 mit anschließender Integration in das Genom wurde der Vektor zunächst mit SacI (Roche Diagnostics GmbH) linearisiert. Die Transformation wurde mittels Elektroporation mit dem Gene Pulser II (Biorad) durchgeführt. Dazu wurde eine Kolonie von Pichia pastoris 5 ml YPD-Medium (Invitrogen) angeimpft und bei 30°C über Nacht unter Schütteln inkubiert. Die Übernachtkultur wurde anschließend 1:2000 in 200 ml frisches YPD-Medium (Invitrogen) überimpft und über Nacht bei 30°C unter Schütteln inkubiert, bis eine $OD_{600}$ von 1,3 bis 1,5 erreicht war. Die Zellen wurden abzentrifugiert (1500 x g / 5 Minuten) und das Pellet in 200 ml eiskaltem, sterilen Wasser (0°C) resuspendiert. Die Zellen wurden wiederum abzentrifugiert (1500 x g/5 Minuten) und in 100 ml eiskaltem, sterilen Wasser (0°C) resuspendiert. Die Zellen wurden wiederum abzentrifugiert, in 10 ml eiskaltem (0°C) 1 M Sorbitol (ICN) resuspendiert. Die Zellen wurden wiederum abzentrifugiert, in 0,5 ml eiskaltem (0°C) 1 M Sorbitol (ICN) resuspendiert. Die so gewonnenen Zellen wurden auf Eis gehalten und sofort zur Transformation eingesetzt.

80 µl der Zellen wurden mit ca. 1 µg linearisierter pHAP10-3-Vektor-DNA versetzt und der gesamte Ansatz in eine eiskalte (0°C) Elektroporationsküvette transferiert und weitere 5 Minuten auf Eis inkubiert. Anschließend wurde die Küvette in den Gene Pulser II (Biorad) überführt und die Transformation bei 1 kV, 1 kΩ und 25µF durchgeführt. Nach der Elektroporation wurde der Ansatz mit 1 ml 1M Sorbitol (ICN) versetzt und anschließend 100 bis 150 µl auf eine YPDS-Agarplatte (Invitrogen) mit 100µg/ml Zeocin® (Invitrogen) ausplattiert. Die Platten wurden anschließend 2 - 4 Tage bei 30 °C inkubiert.

Die Klone wurden auf Raster-MD (= minimal Dextrose)-Platten überimpft und weiter analysiert. Gewachsene Klone wurden gepickt, in 20 µl steriles Wasser resuspendiert, mit 17,5 U Lyticase (Roche Diagnostics GmbH) aufgeschlossen (1 Stunde, 37°C) und direkt mittels PCR auf die korrekte Integration der synthetischen AP-Expressionskassette untersucht.

Klone, die die komplette Expressionskassette bei der Transformation in das Genom integriert haben, wurden dann in Expressionsversuchen eingesetzt.

Expression der hochaktiven Alkalischen Phosphatase

Positive Klone wurden in 3 ml BMGY-Medium (Invitrogen) angeimpft und über Nacht bei 30°C unter Schütteln inkubiert. Anschließend wurden die OD bei 600 nm bestimmt und so in 10ml BMMY-Medium (Invitrogen) überimpft, daß

eine $OD_{600}$ von 1 resultierte. Das BMMY-Medium (Invitrogen) enthält Methanol (Mallinckrodt Baker B.V.), das die Expression der hochaktiven Alkalischen Phosphatase über den AOX 1-Promotor induziert.

Die Schüttelkolben wurden bei 30°C unter Schütteln inkubiert, alle 24 Stunden Proben gezogen, die $OD_{600}$ bestimmt, ein Aktivitätstest auf Expression der hochaktiven Alkalischen Phosphatase durchgeführt und jeweils mit 0,5% Methanol (Mallinckrodt Baker B.V.) zur weiteren Induktion nachgefüttert. Die Expressionsversuche liefen über 96 Stunden.

Test auf Aktivität der hochaktiven Alkalischen Phosphatase

Je 500 µl wurden der Expressionskultur entnommen, die $OD_{600}$ bestimmt und die Zellen abzentrifugiert. Der Überstand wurde aufbewahrt und das Zellpellet wurde in einer der $OD_{600}$ entsprechenden Menge Y-PER™ (50 bis 300 µl/Pierce) zur Lyse resuspendiert und 1 Stunde bei Raumtemperatur geschüttelt. Anschließend wurde das Lysat zur Abtrennung von Zelltrümmern abzentrifugiert (15000 x g/5 Minuten) und der Überstand in frische Reaktionsgefäße überführt. 5 µl des Lysates wurden dann im Aktivitätstest eingesetzt.

Der Aktivitätstest wurde wie oben beschrieben durchgeführt.

Beispiel 4:

Steigerung der Expressionsleistung durch Mehrfachtransformation

Die besten Klone aus den Expressionsversuchen wurden wiederum für die Elektroporation wie oben beschrieben vorbereitet und wiederum mit 1 µg linearisierter pHAP10-3-Vektor-DNA transformiert und der Transformationsansatz auf YPDS-Agarplatten (Invitrogen) mit 1000 bis 2000 µg/ml Zeocin® (Invitrogen) ausplattiert. Dadurch wird der Selektionsdruck derart erhöht, daß nur Klone wachsen können, die mehrere Kopien des Expressionsvektors pHAP10-3 und somit auch mehrere Kopien jeweiligen Resistenzgens (hier Zeocin®) in das Genom integriert haben. Das Zeocin®-Resistenzprotein ist das Produkt des Bleomycingens von Streptoalloteichus hindusstanus (Chalmels, T. et al., Curr. Genet. 20 (1991), 309-314; Drocourt, D. et al., Nucleic Acid Research 18 (1990), 4009), das Zeocin® in einem stöchiometrischen Konzentrationsverhältnis bindet und somit der Zelle Resistenz gegenüber Zeocin® verleiht. Je höher die Konzentration an Zeocin® in den YPDS-Agarplatten, um so mehr Resistenzprotein muß die Zelle erzeugen, um das Zeocin® quantitativ zu binden und damit ein Wachstum zu ermöglichen. Dies ist u.a. möglich, wenn multiple Kopien des Resistenzgens in das Genom integriert werden. Klone wurden wie oben beschrieben auf Raster-MD-Platten überimpft und wiederum wie oben beschrieben mittels PCR-Analyse auf korrekte Integration der haAP-Expressionskassette überprüft. Anschließend wurden diese Klone wiederum wie oben beschrieben auf haAP-Aktivität getestet.

Beispiel 5:

Steigerung der Expressionsleistung durch Verwendung eines zweiten Selektionsdruckes

Eine Steigerung der Zeocin®-Konzentration über 2000 µg/ml führte nicht zu einer verbesserten Expressionsleistung der hochaktiven Alkalischen Phosphatase. Um die Genkopienzahl des Gens gemäß SEQ ID NO: 5, das für die hochaktive Alkalische Phosphatase kodiert und für die Expression in Hefe codon-optimiert ist, in den Expressionsklonen weiter zu erhöhen, wurde die Integration zusätzlicher Expressionsvektoren in das Genom der aus Beispiel 3 und 4 hervorgegangenen Expressionsklone mit der höchsten Expressionsleistung über einen zweiten Selektionsdruck, bevorzugt G418 (Roche Diagnostics GmbH) selektioniert. Zu diesem Zweck wurde die gesamte Expressionskassette aus pHAP10-3, bestehend aus AOX 1-Promotor, Signalpeptid des α-Faktors aus Saccharomyces cerevisiae, codon-optimiertes Gen für die hochaktive Alkalische Phosphatase gemäß SEQ ID NO: 5 und AOX 1-Transkriptionsterminations-Region, mittels PCR durch entsprechend gewählte Primer isoliert wie unten beschrieben in den Vektor pPIC9K kloniert, dessen Integration in das Genom von Pichia pastoris über G418 (Roche Diagnostics GmbH) selektioniert wird. Die Primer sind in SEQ ID NO: 34 und 35 dargestellt.

Der PCR-Ansatz wurde mittels Agarosegelelektrophorese analysiert, das Genfragment mit der erwarteten Größe isoliert (QIAquick Gel Extraction Kit/Qiagen), mit SacI und NotI (Roche Diagnostics GmbH) nachgeschnitten, anschließend wieder aus dem Agarosegel isoliert (QIAquick Gel Extraction Kit/Qiagen) und in ein ebenfalls mit SacI/NotI (Roche Diagnostics GmbH) linearisiertes und isoliertes Vektorfragment von pPIC9K ligiert. Somit war gewährleistet, daß die gesamte Expressionskassette aus pHAP10-3 identisch in pPIC9K vorlag. Das insertierte Fragment wurde mittels Restriktionsanalyse und Sequenzierung mit den flankierenden Regionen überprüft. Der so entstandene Expressionsvektor wurde pHAP10-3/9K (s. Fig. 3) genannt.

Die Klone mit der höchstens haAP-Expressionsleistung aus der Mehrfachtransformation mit pHAP10-3 (Zeocinresistenz) wurden wie oben beschrieben für die Elektroporation vorbereitet und mit 1 µg mit SacI (Roche Diagnostics

GmbH) linearisierten Vektorfragment von pHAP10-3/9K wie oben beschrieben transformiert. Der Transformationsansatz wurde anschließend 1 bis 3 Tage bei 4°C in 1 M Sorbitol (ICN) aufbewahrt (zur Ausbildung der G418-Resistenz) und dann 100 bis 200 µl auf YPD-Platten (Invitrogen) mit 1, 2 bzw. 4 mg/ml G418 (Roche Diagnostics GmbH) ausplattiert und 3 bis 5 Tage bei 30°C inkubiert. Daraus resultierende Klone wurden wiederum wie beschrieben über den Aktivitätstest auf eine erhöhte Expression der eukaryontischen hochaktiven Alkalischen Phosphatase untersucht.

**Patentansprüche**

1. Transformierter Wirtsstamm, erhältlich durch die Durchführung der Schritte

   (a) Klonieren einer codon-optimierten Gensequenz codierend für eine eukaryontische Alkalische Phosphatase in jeweils einem ersten Expressionsvektor mit einem Resistenzgen gegen ein erstes Antibiotikum und einem zweiten Expressionsvektor mit einem Resistenzgen gegen ein zweites Antibiotikum; gefolgt von
   (b) Transformieren eines Hefe-Wirts mit dem ersten Expressionsvektor und Selektion von Transformanten, die mindestens eine Kopie des ersten Expressionsvektors mit der Gensequenz und dem Resistenzgen gegen das erste Antibiotikum in das Genom integriert haben, durch Wachstum auf Nährmedium mit einer geringen Konzentration des ersten Antibiotikums; gefolgt von
   (c) Erhöhen der Genkopienzahl des ersten Expressionsvektors durch Mehrfachtransformation mit dem ersten Expressionsvektor, wobei durch Wachstum auf Nährmedium mit einer im Vergleich zu Schritt (b) höheren Konzentration des ersten Antibiotikums selektioniert wird; gefolgt von
   (d) Transformieren von transformierten Klonen des Wirts mit der höchsten Expressionsleistung an Alkalischer Phosphatase mit dem zweiten Expressionsvektor und Selektion von Transformanten, die neben den Kopien des ersten Expressionsvektors Kopien des zweiten Expressionsvektors mit der Gensequenz und dem Resistenzgen gegen das zweite Antibiotikum aufgenommen haben, durch Wachstum auf Nährmedium mit dem zweitem Antibiotikum; gefolgt von;
   (e) Erhöhen der Genkopienzahl des zweiten Expressionsvektors durch Mehrfachtransformation mit dem zweiten Expressionsvektor, wobei durch Wachstum auf Nährmedium mit einer im Vergleich zu Schritt (d) höheren Konzentration des zweiten Antibiotikums selektioniert wird; gefolgt von
   (f) Selektionieren der Klone, die mehrere Kopien der Gensequenz und der Selektionsmarker-Resistenzgene stabil in das Genom integriert haben; und
   (g) Untersuchen der in Schritt (f) erhaltenen Klone auf erhöhte Expression durch Aktivitätstest der Alkalischen Phosphatase;

   wobei die Expressionsleistung der in Schritt (f) erhaltenen Klone um den Faktor 4 gegenüber der Expression der in Schritt (b) erhaltenen Klone gesteigert ist.

2. Transformierter Wirtsstamm nach Anspruch 1, **dadurch gekennzeichnet, dass** die von dem ersten und dem zweiten Expressionsvektor enthaltene codon-optimierte Gensequenz SEQ ID NO:1 mit stummen Mutationen ist.

3. Transformierter Wirtsstamm nach Anspruch 2, **dadurch gekennzeichnet, dass** die von dem ersten und dem zweiten Expressionsvektor enthaltene codon-optimierte Gensequenz SEQ ID NO:5 ist.

4. Transformierter Wirtsstamm nach Anspruch 3, **dadurch gekennzeichnet, dass** die codon-optimierte Gensequenz durch de novo Synthese bereitgestellt wird.

5. Transformierte Wirtsstamm nach einem der Ansprüche 1 bis 4, **dadurch gekennzeichnet, dass** der Hefe-Wirt Pichia pastoris oder Hansenula polymorpha ist.

6. Transformierter Wirtsstamm nach Anspruch 5, **dadurch gekennzeichnet, dass** der Hefe-Wirt der Pichia pastoris Stamm X33 ist.

7. Transformierter Wirtsstamm nach einem der Ansprüche 5 und 6, **dadurch gekennzeichnet, dass** das Produkt des Bleomycingens von Streptoalloteichus hindustanus das Produkt des Resistenzgens des ersten Expressionsvektors ist.

8. Transformierter Wirtsstamm nach Anspruch 7, **dadurch gekennzeichnet, dass** das erste Antibiotikum Zeocin® ist.

9. Transformierter Wirtsstamm nach einem der Ansprüche 5 bis 8, **dadurch gekennzeichnet, dass** das zweite Antibiotikum G418 ist.

10. Transformierter Wirtsstamm nach einem der Ansprüche 1 bis 9, **dadurch gekennzeichnet, dass** die Gensequenz in einer Expressionskassette im korrekten Leserahmen hinter der Sequenz codierend das Signalpeptid des alpha-Faktors aus Saccharomyces cerevisiae vorliegt.

11. Transformierter Wirtsstamm nach Anspruch 10, **dadurch gekennzeichnet, dass** die Expressionskassette den AOX 1-Promotor und die AOX 1-Transkriptionsterminationsregion enthält.

12. Transformierter Wirtsstamm nach einem der Ansprüche 1 bis 11, **dadurch gekennzeichnet, dass** der Wirtsstamm hochaktive Alkalische Phosphatase exprimiert und glykosyliert.

13. Flüssigkultur in einem Kulturbehälter enthaltend ein Nährmedium, Methanol und einen transformierten Wirtsstamm nach Anspruch 11.

14. Biomasse enthaltend einen transformierten Wirtsstamm nach Anspruch 12.

## Claims

1. Transformed host strain obtainable by carrying out the steps:

(a) Cloning a codon-optimized gene sequence coding for a eukaryotic alkaline phosphatase in in each case a first expression vector with a resistance gene against a first antibiotic and a second expression vector with a resistance gene against a second antibiotic; followed by
(b) transforming a yeast host with the first expression vector and selecting transformants which have integrated at least one copy of the first expression vector with the gene sequence and the resistance gene against the first antibiotic into the genome, by growth on a nutrient medium containing a low concentration of the first antibiotic; followed by
(c) increasing the gene copy number of the first expression vector by multiple transformation with the first expression vector and selecting by growth on nutrient medium containing a higher concentration of the first antibiotic compared to step (b); followed by
(d) transforming transformed clones of the host with the highest alkaline phosphatase expression efficiency with the second expression vector and selecting for transformants which, in addition to copies of the first expression vector have integrated copies of the second expression vector with the gene sequence and the resistance gene against the second antibiotic, by growth on nutrient medium containing the second antibiotic; followed by
(e) increasing the gene copy number of the second expression vector by multiple transformation with the second expression vector and selecting by growth on nutrient medium containing a higher concentration of the second antibiotic compared to step (d); followed by
(f) selecting clones which have stably integrated several copies of the gene sequence and the selection marker resistance genes into the genome; and
(g) examining the clones obtained in step (f) for increased expression by an activity test for alkaline phosphatase;

wherein the expression efficiency of the clones obtained in step (f) is increased four-fold compared to the expression of the clones obtained in step (b).

2. Transformed host strain according to claim 1, **characterized in that** the codon-optimized gene sequence containing the first and the second expression vector is SEQ ID NO:1 with silent mutations.

3. Transformed host strain according to claim 2, **characterized in that** the codon-optimized gene sequence containing the first and the second expression vector is SEQ ID NO:5.

4. Transformed host strain according to claim 3, **characterized in that** the codon-optimized gene sequence is prepared by de novo synthesis.

5. Transformed host strain according to one of the claims 1 to 4, **characterized in that** the yeast host is Pichia pastoris or Hansenula polymorpha.

6. Transformed host strain according to claim 5, **characterized in that** the yeast host is the Pichia pastoris strain X33.

7. Transformed host strain according to one of the claims 5 and 6, **characterized in that** the product of the bleomycin gene of Streptoalloteichus hindustanus is the product of the resistance gene of the first expression vector.

8. Transformed host strain according to claim 7, **characterized in that** the first antibiotic is Zeocin®.

9. Transformed host strain according to one of the claims 5 to 8, **characterized in that** the second antibiotic is G418.

10. Transformed host strain according to one of the claims 1 to 9, **characterized in that** the gene sequence is present in an expression cassette in the correct reading frame downstream of the sequence coding for the signal peptide of the alpha factor from Saccharomyces cerevisiae.

11. Transformed host strain according to claim 10, **characterized in that** the expression cassette contains the AOX 1 promoter and the AOX 1 transcription termination region.

12. Transformed host strain according to one of the claims 1 to 11, **characterized in that** the host strain expresses and glycosylates highly active alkaline phosphatase.

13. Liquid culture in a culture container containing a nutrient medium, methanol and a transformed host strain according to claim 11.

14. Biomass containing a transformed host strain according to claim 12.

## Revendications

1. Souche hôte transformée, pouvant être obtenue par la réalisation des étapes

(a) clonage d'une séquence génique optimisée en ce qui concerne les codons, codant pour une phosphatase alcaline eucaryote dans à chaque fois un premier vecteur d'expression présentant un gène de résistance à un premier antibiotique et un deuxième vecteur d'expression présentant un gène de résistance à un deuxième antibiotique ; suivi d'une
(b) transformation d'une levure-hôte avec le premier vecteur d'expression et sélection de transformants qui ont intégré au moins une copie du premier vecteur d'expression présentant la séquence génique et le gène de résistance au premier antibiotique dans le génome, par croissance sur du milieu nutritif présentant une faible concentration en premier antibiotique ; suivie d'une
(c) augmentation du nombre de copies du gène du premier vecteur d'expression par transformation multiple avec le premier vecteur d'expression, en sélectionnant, par la croissance sur du milieu nutritif, ayant concentration en premier antibiotique plus élevée par rapport à l'étape (b) ; suivie d'une
(d) transformation de clones transformés de l'hôte présentant la puissance d'expression la plus élevée de phosphatase alcaline avec le deuxième vecteur d'expression et sélection de transformants qui ont intégré, outre les copies du premier vecteur d'expression, des copies du deuxième vecteur d'expression présentant la séquence génique et le gène de résistance au deuxième antibiotique, par croissance sur du milieu nutritif contenant le deuxième antibiotique ; suivie d'une
(e) augmentation du nombre de copies du gène du deuxième vecteur d'expression par transformation multiple avec le deuxième vecteur d'expression, en sélectionnant, par la croissance sur du milieu nutritif, ayant concentration en deuxième antibiotique plus élevée par rapport à l'étape (d) ; suivie d'une
(f) sélection des clones qui ont intégré de manière stable dans le génome plusieurs copies de la séquence génique et des gènes de résistance au marqueur de sélection ; et
(g) analyse de l'expression augmentée des clones obtenus dans l'étape (f) par le test d'activité de la phosphatase alcaline ;

la puissance d'expression des clones obtenus dans l'étape (f) étant augmentée d'un facteur 4 par rapport à l'expression des clones obtenus dans l'étape (b).

2. Souche hôte transformée selon la revendication 1, **caractérisée en ce que** la séquence génique optimisée en ce qui concerne les codons obtenue du premier et du deuxième vecteur d'expression est la séquence SEQ ID NO : 1 avec des mutations silencieuses.

3. Souche hôte transformée selon la revendication 2, **caractérisée en ce que** la séquence génique optimisée en ce qui concerne les codons obtenue du premier et du deuxième vecteur d'expression est la séquence SEQ ID NO : 5.

4. Souche hôte transformée selon la revendication 3, **caractérisée en ce que** la séquence génique optimisée en ce qui concerne les codons est préparée par synthèse de novo.

5. Souche hôte transformée selon l'une quelconque des revendications 1 à 4, **caractérisée en ce que** la levure-hôte est Pichia pastoris ou Hansenula polymorpha.

6. Souche hôte transformée selon la revendication 5, **caractérisée en ce que** la levure-hôte est la souche X33 de Pichia pastoris.

7. Souche hôte transformée selon l'une quelconque des revendications 5 et 6, **caractérisée en ce que** le produit du gène de la bléomycine de Streptoalloteichus hindustanus est le produit du gène de résistance du premier vecteur d'expression.

8. Souche hôte transformée selon la revendication 7, **caractérisée en ce que** le premier antibiotique est la Zeocine®.

9. Souche hôte transformée selon l'une quelconque des revendications 5 à 8, **caractérisée en ce que** le deuxième antibiotique est le G418.

10. Souche hôte transformée selon l'une quelconque des revendications 1 à 9, **caractérisée en ce que** la séquence génique se trouve dans une cassette d'expression dans le cadre de lecture correct en aval de la séquence codant pour le peptide signal du facteur alpha de Saccharomyces cerevisiae.

11. Souche hôte transformée selon la revendication 10, **caractérisée en ce que** la cassette d'expression contient le promoteur AOX 1 et la région de fin de transcription AOX 1.

12. Souche hôte transformée selon l'une quelconque des revendications 1 à 11, **caractérisée en ce que** la souche hôte exprime et glycosyle la phosphatase alcaline hautement active.

13. Culture liquide dans un récipient de culture contenant un milieu nutritif, du méthanol et une souche hôte transformée selon la revendication 11.

14. Biomasse contenant une souche hôte transformée selon la revendication 12.

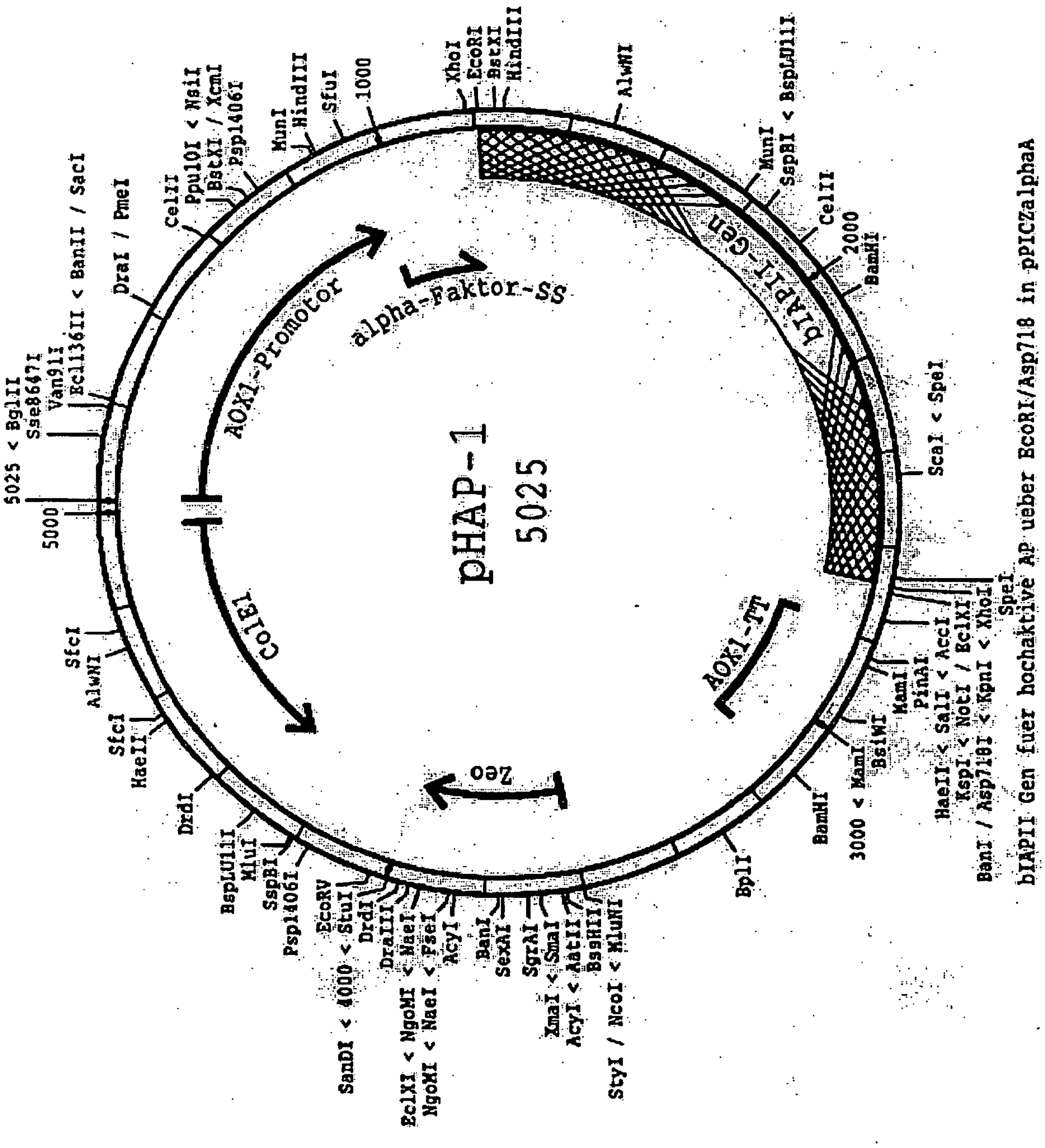
pHAP-1
5025
AOX1-Promotor
alpha-Faktor-SS
bIAPII-Gen
AOX1-TT
Zeo
ColE1
XhoI
EcoRI
BstXI
HindIII
AlwNI
MunI
SspBI < BspLU11I
CelII
2000
BamHI
ScaI < SpeI
SpeI
3000 < MamI
BamHI
BplI
1000
SfuI
MunI
HindIII
Psp1406I
BstXI / XcmI
Ppu10I < NsiI
CelII
DraI / PmeI
Ecl136II < BanII / SacI
Van91I
Sse8647I
5025 < BglII
5000
SfcI
AlwNI
SfcI
HaeII
DrdI
BspLU11I
MluI
SspBI
Psp1406I
EcoRV
SanDI < 4000 < StuI
DrdI
DraIII
BanI
SexAI
SgrAI
StyI / NcoI < MluNI
bIAPII Gen fuer hochaktive AP ueber EcoRI/Asp718 in pPICZalphaA

Fig.1

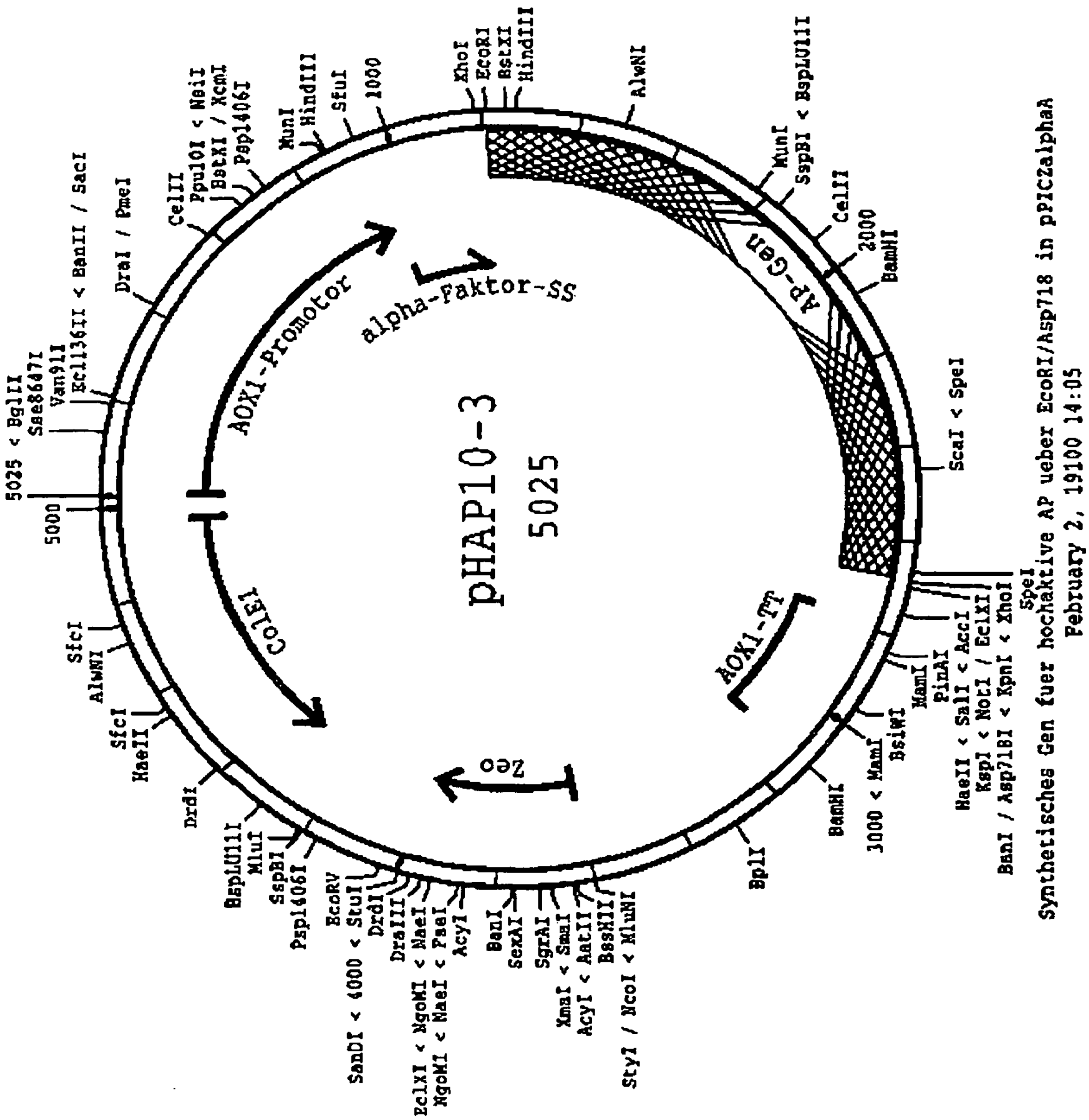
pHAP10-3
5025
AOX1-Promotor
alpha-Faktor-SS
Ap-Gen
AOX1-TT
Zeo
ColE1
Synthetisches Gen fuer hochaktive AP ueber EcoRI/Asp718 in pPICZalphaA
February 2, 19100 14:05

Fig. 2

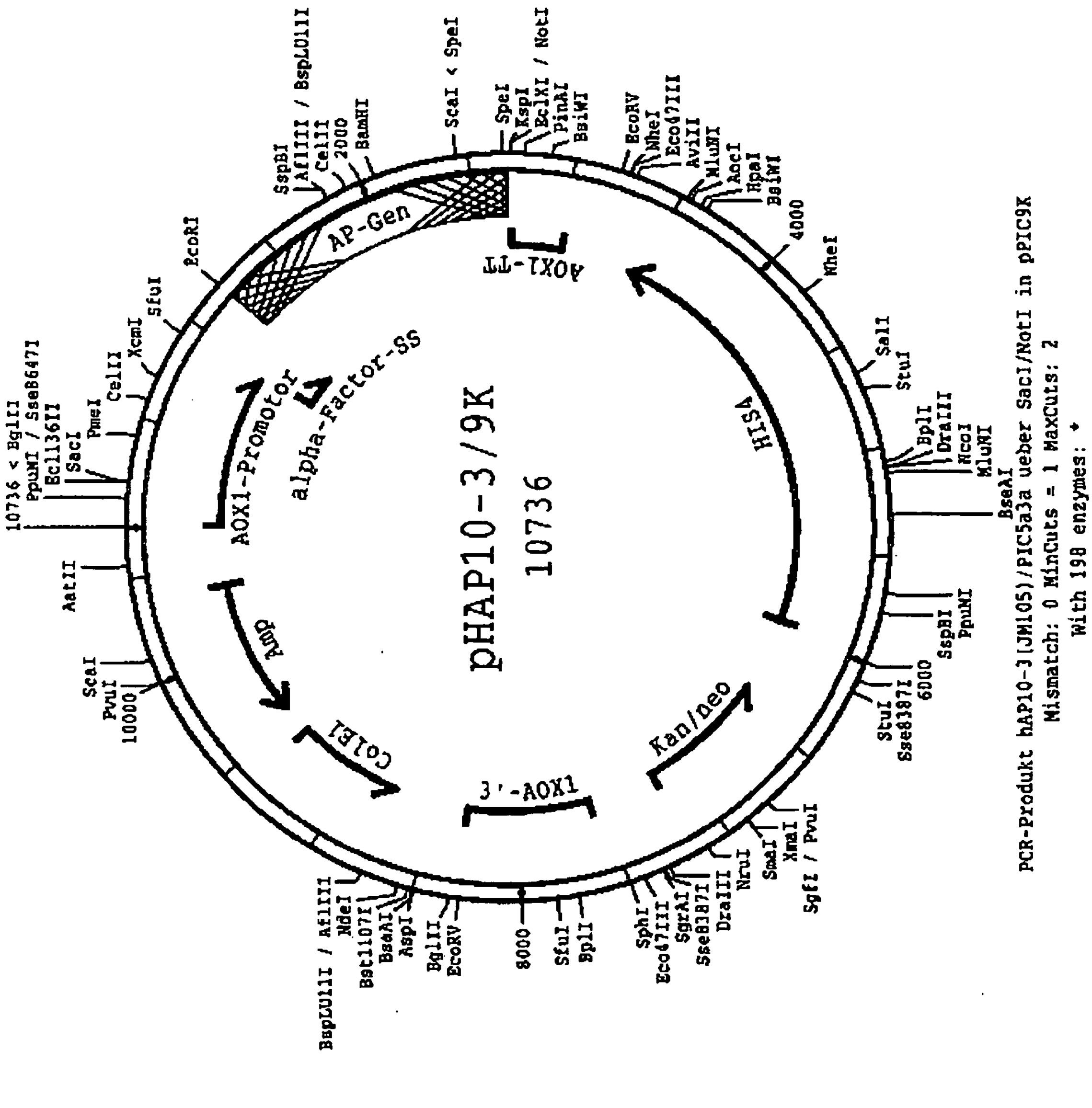
pHAP10-3/9K
10736
AOX1-Promotor
alpha-Factor-SS
AP-Gen
AOX1-TT
HIS4
Kan/neo
3'-AOX1
ColEI
Amp
PCR-Produkt hAP10-3(JM105)/PIC5a3a ueber SacI/NotI in pPIC9K
Mismatch: 0 MinCuts = 1 MaxCuts: 2
With 198 enzymes: *

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0955369 A
- WO 9318139 A
- DE 19819962


### In der Beschreibung aufgeführte Nicht-Patentliteratur


- **McComb et al.** Alkaline Phosphatases. Plenum Press, 1979
- **Millàn.** *Anticancer Res.,* 1988, vol. 8, 995-1004
- **Harris.** *Clin. Chim. Acta,* 1989, vol. 186, 133-150
- **Millan.** *Anticancer Res.,* 1988, vol. 8, 995-1004
- **Manes et al.** *J. Biol. Chem.,* 1998, vol. 273 (36), 23353-23360
- **Weissig et al.** *Biochem J.,* 1993, vol. 260, 503-508
- **Berger et al.** *Biochemistry,* 1987, vol. 84, 4885-4889
- **Davis et al.** *Biotechnology,* 1992, vol. 10, 1148-1150
- **Burtscher.** *Protein Expression and Purification,* 1994, vol. 5, 192-197
- **Cregg, J.M. et al.** *Mol. Cell. Biol.,* 1985, vol. 5, 3376-3385
- **Manes et al.** *J. Biol. Chem,* 1998, vol. 273 (36), 23353-23360
- **Chalmels, T. et al.** *Curr. Genet.,* 1991, vol. 20, 309-314
- **Drocourt, D. et al.** *Nucleic Acid Research,* 1990, vol. 18, 4009